# EUROPEAN PATENT SPECIFICATION

(11) **EP 2 281 552 B1**
(45) Date of publication and mention of the grant of the patent: **09.05.2012**
(21) Application number: 09733479.1
(22) Date of filing: 18.02.2009
(51) Int. Cl.: A61K 8/92, A61K 8/97, A61Q 19/00, C11C 5/00

(54) **CANDLE FOR MASSAGE, METHOD FOR OBTAINING MASSAGE OIL, AND MASSAGE METHOD**
MASSAGEKERZE, VERFAHREN ZUR GEWINNUNG VON MASSAGEÖL DARAUS UND MASSAGEVERFAHREN
BOUGIE POUR MASSAGE, PROCÉDÉ D'OBTENTION D'UNE HUILE DE MASSAGE ET PROCÉDÉ DE MASSAGE

(30) Priority: 17.04.2008 WO PCT/JP2008/057482
(43) Date of publication of application: 09.02.2011
(73) Proprietor: Iijima, Noriko, Kanagawa 248-0035 (JP)
(72) Inventor: Iijima, Noriko, Kanagawa 248-0035 (JP)
(74) Representative: Graf von Keyserlingk, Albert
(86) International application number: PCT/JP2009/000685
(87) International publication number: WO 2009/128192

(56) References cited:
- WO-A2-2006/023906
- WO-A2-2006/028533
- US-A1- 2006 042 157

## Description

### TECHNICAL FIELD

The present invention relates to a candle for massage.

### BACKGROUND OF ART

Massage oil are sometimes used when executing massage. Using the oil for massage makes fingers of an administrator smooth and it leads massage to be effective. The oil for massage has a variety of effects to skin. Massage oil is usually used in room temperature. Thus someone who is administered massage sometimes feels that oil is cold. To prevent this it may be thought that the oil should be heated and an administrator should massage using the heated oil. In this case, it requires equipment for heating and it may cause accidents. Further, the oil temperature must be controlled so as not to cause burns or scales.

Patent Publication No. 2005-194408 (Patent Document 1) discloses massage oil that comprises an essential oil made from the loquat. The paragraph [0017] of the document discloses carrier oil. However the massage oil and carrier oils are liquid.

Patent Publication No. 2000-351712 (Patent Document 2) discloses cosmetic that can give feeling as if it were sorbet. The paragraph [0036] of the document discloses a method for manufacturing of cosmetics for massage. However, these cosmetics were felt as if it were a sorbet and is not intended to be heated.

Patent Publication No. 2003-41087 (Patent Document 3) discloses gel composition. The paragraph [0059] discloses examples of natural fats and natural oils. In addition, paragraph [0126] and [0137] discloses that gel composition may be used for beauty. However, this document is not intended to use the gel composition for massage.
[Patent Document 1] Patent Publication No. 2005-194408
[Patent Document 2] Patent Publication No. 2000-351712
[Patent Document 3] Patent Publication No. 2003-41087

### DISCLOSURE OF INVENTION

### PRPBLEMS TO BE SOLVED BY THE INVENTION

An object of the present invention is to provide a candle for massage that can have a suitable temperature for massage.
Other object of the present invention is to provide a candle for massage that can strengthen the permeability of the components contained in oil because the massage oil is warmed.
Other object of the present invention is to provide a candle for massage that can make active ingredients effective and can make the effect appear promptly.

It is another object of the present invention to provide a candle for massage that has excellent appearance so that users feel exiting and regard it luxurious and massage becomes effective.

It is still another object of the present invention to provide a method for manufacturing massage oil for massage using the above described candle for massage and a method for administrating massage.

### MEANS FOR SOLVING THE PROBLEM

Previously, there is no cosmetic for massage the shape of which is candle-like. Thus the candle for massage of the present invention can make a person who is administered a massage feel excited and feel luxurious and it leads the massage effective. Further, the present invention can obtain massage oil that has suitable temperature by igniting a candle for massage the melting point of which is adjusted.

The first aspect of the invention directed to a candle for massage. The candle for massage of the present invention contains a container 2; a wax component 3 which is contained in the container 2; and a wick 4 which is inserted into the wax component 3. To our knowledge, there was no candle for massage previously. Thus the candle for massage of the present invention can make a person who is administered a massage feel excited and feel luxurious and it leads the massage effective and it can make massage effective.

It is preferred that the container 2 has a spout 5 so that dissolved wax component is poured from the spout. If the candle contains the spout 5 it is easy to pour oil which is the melted wax.

It is preferred that the wax component 3 comprises solid fat and liquid oil and the melting point of the wax component is from 30 degrees Celsius to 60 degrees Celsius. When the melting point of the wax component is the above scope, the melted oil can provide comfortable warmness with a person who is administered massage without the risk of burns.

It is preferred that the wax component 3 comprises 40 wt % or more of the solid fat and from 15 wt % to 40 wt % of liquid oil. By adjusting the weight ratio of solid fat and liquid oil in this way, the melting point of the wax component can be controlled.

It is preferred that the solid fat is a solid vegetable fat. The preferred solid oil comprises shea butter. Further, the liquid oil is preferred to be one or a plurality of vegetable oils. A preferred wax component comprises palm oil.

It is preferred that the wax component 3 further comprises from 2 wt % to 15 wt% of thickeners. When the wax component 3 comprises thickeners it is easy to produce composition of wax component effectively.

The above described features may be used in any combination.

The second aspect of the present invention is directed to a method for obtaining oil for massage. The method fundamentally relates to a method for obtaining oil by using the above described candle for massage of the present invention. The method comprises a step of providing a candle for massage, a step of igniting the wick 4; and a step of melting the wax component 3 to obtain liquid oil. Then the method may bring oil for massage that has a suitable temperature for massage.

A third aspect of the invention relates to a method of massage for beauty using the above described oil of the present invention. This method including a step of administrating massage using liquid oil that is obtained by melting the candle for massage of the present invention.

It is preferred that the step of administrating massage comprises a step of administrating massage along with langer lines.

### EFFECT OF THE INVENTION

The present invention can provide a candle for massage that can bring a suitable temperature for massage. The permeability of the components contained in oil is strengthened because the massage oil is warmed to a suitable temperature. Thus the oil can be used effectively and promptly. Thus an administrator can massage smoothly. The person who is administered massage may feel comfortable because the oil is warmed at a suitable temperature and effect of ingredients that are comprised in the oil appears promptly. The candle for massage of the present invention may bring satisfaction to both an administrator and a person who is administered massage.

A candle for massage of the present invention can has excellent appearance and it makes users feel exiting and regard it luxurious and massage becomes effective.

The present invention can provide a method for manufacturing massage oil for massage using the above described candle for massage and a method for administrating massage.

### BEST MODE FOR CARRYING OUT THE INVENTION

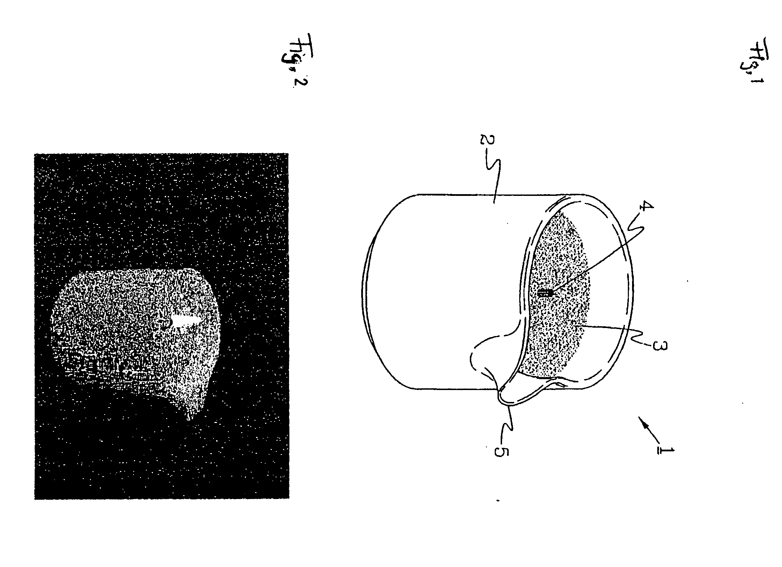

The embodiments of the present invention are disclosed hereafter. Figure 1 is a conceptual diagram that shows a candle for massage of the present invention. As shown in Figure 1, the candle for massage of the invention contains a container 2; a wax component 3 which is contained in the container 2; and a wick 4 which is inserted into the wax component 3

The container or the vessel of the Figure 1 has a beaker-like shape. However, the shape of container 2 may not be limited to be beaker like shape. It is preferred that the container 2 has a spout 5 so that dissolved wax component is poured from the spout. The spout may be set at the upper side of the container. If the candle contains the spout 5 it is easy to pour oil of the melted wax. It is preferred that the container is heatproof or has a character of heat resistance. An example of the container 2 is a container of ceramics. Container 2 may be plastic container. The size of the container 2 may be adjusted based on the usage of the candle. The example of the size of the container 2 is from 10 ml to I liter, and may be from 50 ml to 500 ml.

The wax component 3 conforms the candle body. When the wick 4 is ignited, the wax component 3 melts and a part of the wax component 3 is consumed to keep flame.

It is preferred that the wax component 3 comprises solid fat and liquid oil. It is preferred that the melting point of the wax component is from 30 degrees Celsius to 60 degrees Celsius. It is still preferred that the melting point of the wax component is from 35 degrees Celsius to 55 degrees Celsius and it may be from 35 degrees Celsius to 50 degrees Celsius.
It is possible to arrange the melting point of the wax component 3 by adjusting kind of solid fat and liquid oil, and the ratio thereof. For example when higher fatty acid that has melting point of more than 50 degrees Celsius is added, the melting temperature of the wax component 3 becomes higher. To have above region of the melting point, it is preferred that the wax component 3 comprises 40 wt % or more of the solid fat and from 15 wt % to 40 wt % of liquid oil. The term solid fat is intended to mean the fat that is in a solid state in a room temperature. For example, the fat that has melting point of 40 degrees Celsius or more is regarded as solid fat. The rate of the solid fat is preferred to be 50 wt% or more and may be 60 wt% or more.

As solid fat and liquid oil, harmless one may be suitable. Examples of solid fat and liquid oil are disclosed in Patent Publication No. 2003-41087 (Patent Document 3).

Examples of solid fat are vegetable fat, animal fat, waxes, hydrocarbon oils, fatty acids, and higher alcohols. The wax component 3 may contain one of solid fats. The wax component may contain two or more solid fats.

Examples of solid vegetable fat are Shea butter, cocoa butter, mango seed fat and vegetable waxes. Examples of vegetable waxes are Karubana wax, Ouricury wax, palm wax, Kanderirar wax, sugar cane wax, cotton wax, flax wax, Okochira wax, Pisangu wax, and the Esuparuto wax.

Examples of animal fat are cow wax, mutton wax, horse wax and lard. Example of wax is beeswax in addition to the above-mentioned vegetable waxes. Examples of hydrocarbon oils are petroleum jelly and polyolefin. Examples of higher fatty acid are palmitic acid, stearic acid, myristic acid, behenin acid, lauric acid, myristic acid, and hydrochloric acid and docosa hexa eicosa pentan. Example of higher alcohol is Octyldodecanol.

The solid fat is preferably solid vegetable fat. In particular, the solid fat that contains Shea fat is preferred. The Shea fat is extracted fat, extracts and compounds that includes them obtained from the seed of Shea tree (Butyrospermum Parkii Sapotaceae). Shea butter has properties similar to butter. Thus Shea fat is also called as Shea butter. The Shea butter is in solid state at room temperature, and has melting point form 40 degrees Celsius to 60 degrees Celsius. The Shea butter may not only be Shea fat but also be extracts or oil extracted from Shea tree and compounds that includes them.

Liquid oil means the oil that is in liquid state at room temperature. The example of liquid oil is that it has melting point under 40 degrees Celsius. Examples of liquid oils are vegetable oils, animal oils, hydrocarbon oils, higher fatty acids, higher alcohols, silicone oils, esters, and glycerin and derivatives thereof. The wax component may include a type of liquid oil. The wax component may contain two or more liquid oils. It is preferred that wax component 3 comprise liquid oil at 15 wt% to 40 wt %. The content of liquid oil may be 20 wt% to 30 wt%. The wax component may be without liquid oil. The wax component may comprise liquid oil at 1 wt% to 20 wt %.

Examples of vegetable oils are palm oil, almond oil, palm oil, vegetable oil, Carapa guaianensis seed oil, avocado oil, camellia oil, turtle oil, macadamia nut oil, corn oil, sesame oil, Pashikku oil, wheat germ oil, sasanqua oil, castor oil, linseed oil, safflower oil, cottonseed oil, perilla oil, soybean oil, peanut oil, tee seed oil, kaya oil, rice bran oil, jojoba oil, Kyonin, olive oil, Carot oil, grape seed oil and rapeseed oil.

Examples of animal oils are egg yolk oil and mink oil. Examples of hydrocarbon oils are liquid paraffin, squalene, and squalane. Examples of higher fatty acid are oleic acid, tall oil, and isostearic acid. Examples of higher alcohols are lauryl alcohol, oleyl alcohol, iso stearyl alcohol, and the Octyl dodecanol. Examples of silicone oils are methyl polysiloxane, methylphenylpolysiloxane, methyl hydrogen polysiloxane, and the Dekamechiruporishirokisan. Examples of esters are isopropyl myristate, isopropyl palmitate, hexyl laurate, oleyl oleate, decyl oleate, myristate octyldodecyl, hexyldecyl dimethyloctanoate acid, diethyl phthalate, dibutyl phthalate. The examples of glycerin derivatives are one or more than two kind of glycerin derivatives selected from the group of glycerin, di-glycerin, tri-glycerin, tri-octanoate glycerin, and tori isoparumichin glycerin.

Within the liquid oil, preferred one is palm oil. When the wax component comprises palm oil, the oil obtained when the wax component melts becomes smooth and become extendable. Preferred liquid oil comprises palm oil and other oil or oils other than palm oil.

The preferred wax component 3 comprises thickeners at 2 wt% to 15 wt%. When the wax component 3 comprises thickeners, composition can be produced efficiently using solid fat and liquid oil. Example of thickeners agent is silicic anhydride. As silicic anhydride, silica is preferred that the silylation process.

The wax component 3 may further comprise fragrance. When such a fragrance evaporates, the candle for massage of the present invention can give an effect of aromatherapy. It is possible to use already known fragrances. Examples of fragrance are Angelica, all Nzoin, oregano, orange, chamomile, cajeput, Garubanamu, clary sage, grapefruit, Cypress, Sandalwood, cedarwood, citronella, cinnamon, jasmine, juniper, ginger, Supaikurapenda, spearmint, sage, Zeraniumu, thyme, tangerine, tea tree, nutmeg, frankincense, neroli, pineapple, basil, Patchouli, verbena, rose, palmarosa, fennel, petitgrain, vetiver, peppermint, Rugamotto all, Marjoram, Mandarin, Melissa, Eucalyptus, Rabanjin, vendors labeling lemon, lemongrass, rosewood and rosemary. The wax component wax may contain only one type of fragrance. The wax component may include two or more kinds of fragrances. Examples of the content of the perfume are 100 ppm to 10 wt%, and preferred content of the perfume is 300 ppm to 1 wt%.

The wax component may contain nutrients for skin. The examples of nutrients are vitamins and trehalose. The wax component preferably contains trehalose because trehalose has ability to keep moisture and recover damage.

The wick 4 may be a normal wick that is used to usual candle. The wick 4 may has a part inserted into the wax component 3 portion and the part out of the wax component 3 portion. When the tip of wick 4 which is protruding from the wax component 3 is ignited, the wick can transfer the heat to the wax component, and then the wax component melts by means of the transferred heat. The example of wick is stick like one. When the wick is stick like, the example of the diameter of the stick is from 1mm to 3 mm, and it may be from 1.5 mm to 2.5 mm.

It is possible to manufacture the candle of the present invention using a conventional method to manufacture candles. The conventional method may be arranged using common general knowledge. For example, first, solid fat is heated and then the solid fat melts to be liquid state. Next liquid oil and other ingredients are added and the mixture is stirred. Then it is possible to obtain compound that will be wax component. The liquid compound is poured to a vessel or a container. And then a wick is inserted to the component. After cooling the compound, the wax component becomes solid state. Using this way it is possible to obtain the candle of the present invention.

The second aspect of the present invention is directed to a method for obtaining oil for massage. The method fundamentally relates to a method for obtaining oil by using the above described candle for massage of the present invention. The method comprises a step of providing a candle for massage, a step of igniting the wick 4; and a step of melting the wax component 3 to obtain liquid oil. Then the method may bring oil for massage that has a suitable temperature for massage. The phrase to obtain liquid oil in the step of melting the wax component means to obtain liquid oil such an amount that it is used for massage. The required time to liquefy candle differs based on the ingredient of candle. Examples of time for wax component to be melt is from I second to I minute, preferred example is from 5 seconds to 30 seconds.

When ignite the wick, the wax component below the wick melts by means of the heat from flame at the wick. Because the melting point of the candle is lower than that of usual candle, melted oil accumulates on the top of the wax component.

It is possible to obtain liquid oil by dissolving wax by means of hot water (water bath). However, the obtained oil by using water bath is easy to cool down. Thus it is difficult to obtain enough treatment effect if we obtain liquid oil by means of water bath because the obtained oil cannot keep suitable temperature.

Contrary, the oil obtained by the method of the present invention can keep from 38 degrees Celsius to 42 degrees Celsius, which is suitable for massage, with a long duration. Because the present method liquefies suitable amount of wax component that is required to administrate massage, the method does not consume unnecessary wax component. Further the temperature of the oil is moderate and thus the candle can minimize the evaporation of the essential oils that are melted in the wax component. Further, because heated part is not whole part of the candle, it is possible to minimize the deterioration (eg. oxidation) caused by the heat. It becomes easy for an administrator to execute massage using oil obtained by the method because the obtained oil expands smoothly in a suitable temperature condition. Further, the essential oils and the other ingredients can be absorbed easily because the oil obtained by the present method is in a suitable temperature. Then active ingredients may be absorbed quickly into the blood and then it is possible to utilize the active ingredients. The patient may feel comfortable because the oil is in a comfortable temperature and it is possible to strengthen the effect of massage because active ingredients become efficient quickly.

A third aspect of the invention relates to a method of massage for beauty using the above described oil of the present invention. This method including a step of administrating massage using liquid oil that is obtained by melting the candle for massage of the present invention.

The massage method can make a patient excited and can give a luxurious impression because it uses a new candle of novel form. The patient may feel relaxed because of the visual effect of the flame. When the candle contains fragrance, it can give the effects of aromatherapy to the patient.

In the process of performing a massage, oil for massage may be poured directly to the skin of the patient. Further, the administrator may pick the oil for massage by hand and then she may use the oil for administrating massage. The temperature of oil is basically the melting point of wax component. The temperature of oil does not become higher than the melting point of the wax component before the wax component is completely melted. The preferred temperature of oil is the temperature that does not cause the patient be suffered from burn or scald and patient fells warm. The example of the temperature of the massage oil is from 35 degrees Celsius to 45 degrees Celsius and preferably is from 37 degrees Celsius to 42 degrees Celsius.

It is preferred that the step of administrating massage comprises a step of administrating massage along with langer lines. The langer lines are traces of the cell membrane caused by cell division. Further, the tracks of the langer lines are similar to the tracks of the fibers of the dermis reticular layer of the skin (collagen fibers). Thus it is possible to prevent adding unnecessary power to skin by executing massage along with langer lines. Further it is possible to prevent wrinkles at the skin (dermis and epidermis) and cause crculatory system including blood vessels and lymphatic that are gathered at plexiform layer active. Still further it is possible to make the massage effectively because the essential oils that are masked with the warm oil prevails human body quickly.

### EXAMPLES

Figure 2 shows the manufactured candle for massage is an alternative picture of the drawing. The container is made of porcelain. The size of the container was about the size of a regular cup which is used to drink water.

Table 1 shows the composition of comparative examples and examples. As a comparative example, conventional candles (high melting point), and a candle that has lower melting points were prepared. Silylation treated silicic anhydride was used in the examples as silicic anhydride.

Shea butter, solid fat and liquid oil (fat or oil) except for palm oil used in each examples are as follows:
Example 2: a mixture of palm oil and aloe Bella leaf extract
Example 3: Mango seed fat
Example 4: hydrogenation (hydrogenated) soybean oil
Example 5: a mixture of almond oil and water added vegetable oil.
Example 6: Carapa guaianensis seed oil
Example 7: cocoa butter

The term oil above may include solid fat. Various perfumes were added in each of the working examples and comparative examples. The amount of perfume was several hundred ppm.

**Table 1 Compounds of the wax components in examples and in comparative examples**

| | Examples | | | | | | | | Comparative Examples | |
|---|---|---|---|---|---|---|---|---|---|---|
| | 1 | 2 | 3 | 4 | 5 | 6 | 7 | 9 | 1 | 2 |
| Shea Fat | 60 | 58 | 55 | 52 | 48 | 60 | 56 | 80 | 100 | 40 |
| oil (fat) | - | 20 | 25 | 25 | 30 | 5 | 32 | - | - | - |
| Palm Oil | 28 | 10 | 11 | 12 | 7 | 12 | 5 | - | - | 60 |
| Silicic anhydride | 5 | 5 | 7 | 4 | 7 | 11 | 4 | 5 | - | - |
| Palmitic Acid | 5 | 5 | 1 | 4 | 4 | 7 | 2 | 10 | - | - |
| Steric Acid | - | 2 | 1 | 3 | 4 | 5 | 1 | 5 | - | - |
| Melting Point | 40 | 42 | 41 | 45 | 48 | 51 | 40 | 58 | 82 | 25 |

In Table 1, the numbers except for melting point means weight percent. As a comparative example I, a commercially available candle was used. The conventional candle does not contain Shea butter. When the candle of Comparative Example 1 was used, the oil was so hot that when the oil touched skin we felt that the oil might cause burn or scale. We assessed that the candle of Comparative Example 1 was not fit to massage.

The candle of Comparative Example 2 did not become solid because the melting point of the candle is low. In other words, we could not obtain a candle using the composition of Comparative Example 2.

Candles for massage of Examples 1 to 7 had an appropriate melting point. When the massage oil made by the candles was pasted to the skin, the oil extended well and it was easy to massage.

On the other hand, the candle for massage of Example 8did not extend well and it was not fit to use massage.

### INDUSTRIAL APPLICABILITY

The invention can be suitably used in the cosmetic industry.

### BRIEF DESCRIPTION OF DRAWINGS

Figure 1 shows the present invention is a conceptual diagram showing a candle for massage.
Figure 2 shows the manufactured candle for massage is an alternative picture of the drawing.

### DESCRIPTION OF ELEMENT NUMERALS

- 1: candle for massage
- 2: container
- 3: wax component
- 4: wick
- 5: spout

## Claims

1. A candle for massage comprising:
a container (2);
a wax component (3) which is contained in the container (2); and
a wick (4) which is inserted into the wax component (3),
**characterized in that**,
the wax component (3) comprises solid fat and liquid oil and the melting point of the wax component is from 30 degrees Celsius to 60 degrees Celsius,
the wax component (3) comprises 40 wt % or more of the solid fat and from 15 wt % to 40 wt % of liquid oil, and
the solid fat comprises Shea butter, and the liquid oil comprises vegetable oil.

2. The candle for massage in accordance with claim 1, wherein the container (2) has a spout (5) so that dissolved wax component is poured from the spout.

3. The candle for massage in accordance with claim 1, wherein the wax component (3) further comprises from 2 wt % to 15 wt% of thickeners.

4. The candle for massage in accordance with claim 1,
wherein the wax component (3) comprises from 48 wt % to 80 wt% of shea butter and from 4 wt % to 11 wt % of silicic anhydride

5. The candle for massage in accordance with claim 4, further comprises from 5 wt% to 28 wt% of palm oil.

6. The candle for massage in accordance with claim 5, further comprises 1 wt% to 10 wt% of palmitic acid.

7. The candle for massage in accordance with claim 6, further comprises from 1 wt% to 5 wt% of stearic acid.

8. The candle for massage in accordance with claim 1 or 7, further comprises one or a plurality of essential oils.

9. The candle for massage in accordance with claim 1, wherein the melting point of the wax component is from 40 degrees Celsius to 51 degrees Celsius.

## Patentansprüche

1. Eine Kerze für Massage umfassend:
Einen Behälter (2);
Eine Wachskomponente (3), die im Behälter (2) enthalten ist;
und eine Dochtkomponente (4), welche in die Wachskomponente (3) eingesetzt ist, **gekennzeichnet dadurch, dass** die Wachskomponente (3) ein festes Fett und ein flüssiges Öl umfasst und der Schmelzpunkt der Wachskomponente von 30°C bis 60°C ist, die Wachskomponente (3) 40 Gewichtsprozent oder mehr von dem festen Fett und 15 Gewichtprozent bis 40 Gewichtsprozent von flüssigem Öl umfasst, und
das feste Fett Sheabutter umfasst und das flüssige Öl ein Pflanzenöl umfasst.

2. Kerze nach Anspruch 1, wobei der Behälter (2) eine Tülle (5) aufweist, sodass eine gelöste Wachskomponente aus der Tülle tropfbar ist.

3. Kerze nach Anspruch 1, wobei die Wachskomponente (3) des Weiteren von 2 Gewichtsprozent bis 15 Gewichtsprozent an Festigern umfasst.

4. Kerze nach Anspruch 1, wobei die Wachskomponente (3) von 48 Gewichtsprozent bis 80 Gewichtsprozent von Sheabutter und von 4 Gewichtsprozent zu 11 Gewichtsprozent von einem Silikat Anhydrit umfasst.

5. Kerze nach Anspruch 4, des Weiteren umfassend von 5 Gewichtsprozent zu 28 Gewichtsprozent von Palmöl.

6. Kerze nach Anspruch 5, des Weiteren umfassend 1 Gewichtsprozent bis 10 Gewichtsprozent von Palmitinsäure umfasst.

7. Kerze nach Anspruch 6, des Weiteren umfassend von 1 Gewichtsprozent zu 5 Gewichtsprozent von Stearinsäure.

8. Kerze nach Anspruch 1 oder 7, des Weiteren eine oder eine Vielzahl von essenziellen Fetten umfassend.

9. Kerze nach Anspruch 1, wobei der Schmelzpunkt der Wachskomponente von 40°C bis 51°C ist.

## Revendications

1. Bougie pour massage comprenant :
un récipient (2) ;
un composant en cire (3) qui est contenu dans le récipient (2) ; et
une mèche (4) qui est insérée dans le composant en cire (3),
**caractérisé en ce que**,
le composant en cire (3) est composé de corps gras solide et d'huile liquide et que le point de fusion du composant en cire est compris dans une fourchette de 30 degrés Celsius à 60 degrés Celsius,
le composant en cire (3) comprend 40 % en poids ou plus de corps gras solide et 15 à 40 % en poids d'huile liquide, et
le corps gras solide comprend de beurre de karité et l'huile liquide comprend d'huile végétale.

2. Bougie pour massage selon la revendication 1, dans laquelle le récipient (2) comporte un bec verseur (5) pour que le composant en cire dissous puisse être versé par le bec verseur.

3. Bougie pour massage selon la revendication 1, dans lequel le composant en cire (3) comprend en outre 2 à 15 % en poids d'épaississeurs.

4. Bougie pour massage selon la revendication 1,
dans laquelle le composant en cire (3) comprend 48 à 80 % en poids de beurre de karité et de 4 à 11 % en poids d'anhydride silicique.

5. Bougie pour massage selon la revendication 4, comprenant en outre 5 à 28 % en poids d'huile de palme.

6. Bougie pour massage selon la revendication 5, comprenant en outre 1 à 10 % en poids d'acide palmitique.

7. Bougie pour massage selon la revendication 6, comprenant en outre 1 à 5 % en poids d'acide stéarique.

8. Bougie pour massage selon la revendication 1 ou 7, comprenant en outre d'huile essentielle ou une pluralité d'huiles essentielles.

9. Bougie pour massage selon la revendication 1, dans laquelle le point de fusion du composant en cire est de 40 degrés Celsius à 51 degrés Celsius.
